(19) Europäisches Patentamt
European Patent Office
Office européen des brevets

(11) EP 4 176 881 A1

(12) # EUROPEAN PATENT APPLICATION
published in accordance with Art. 153(4) EPC

(43) Date of publication:
**10.05.2023 Bulletin 2023/19**

(21) Application number: **21850581.6**

(22) Date of filing: **30.07.2021**

(51) International Patent Classification (IPC):
***A61K 31/519*** *(2006.01)* ***A61K 9/08*** *(2006.01)*
***A61K 47/18*** *(2017.01)* ***A61K 47/22*** *(2006.01)*
***A61P 27/02*** *(2006.01)*

(52) Cooperative Patent Classification (CPC):
**A61K 9/08; A61K 31/519; A61K 47/18; A61K 47/22; A61P 27/02**

(86) International application number:
**PCT/JP2021/028441**

(87) International publication number:
**WO 2022/025281 (03.02.2022 Gazette 2022/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.07.2020 JP 2020129730**

(71) Applicant: **Rohto Pharmaceutical Co., Ltd.**
**Osaka-shi, Osaka 544-8666 (JP)**

(72) Inventors:
- **KUBO Ozora**
**Osaka-shi, Osaka 544-8666 (JP)**
- **HAYASHI Saeko**
**Osaka-shi, Osaka 544-8666 (JP)**
- **KITA Akiko**
**Osaka-shi, Osaka 544-8666 (JP)**

(74) Representative: **Mewburn Ellis LLP**
**Aurora Building**
**Counterslip**
**Bristol BS1 6BX (GB)**

(54) **AQUEOUS COMPOSITION**

(57) The present invention relates to an aqueous composition comprising delgocitinib or a salt thereof (A) and at least one selected from the group consisting of edetic acid, creatinine, and a salt thereof (B).

EP 4 176 881 A1

# Description

## Technical Field

The present invention relates to an aqueous composition.

## Background Art

Janus kinase (JAK) is a non-receptor tyrosine kinase that plays an important role in intracellular immunologically activated signal transduction, and drugs having Janus kinase inhibitory activity are expected to improve autoimmune diseases and allergic diseases through suppression of excessive activation of the immune response. As one of the compounds having an inhibitory effect of Janus kinase, 3-[(3S,4R)-3-methyl-6-(7H-pyrrolo[2,3-d]pyrimidin-4-yl)-1,6-diazaspir o[3,4]octan-1-yl]-3-oxopropanenitrile (generic name: delgocitinib) is known (for example, Patent Literature 1).

## Citation List

### Patent Literature

[Patent Literature 1]
International Publication No. WO 2017/006968

## Summary of Invention

### Technical Problem

In the case of using delgocitinib as a therapeutic agent for diseases in the ophthalmic field, the ophthalmic preparation containing delgocitinib is required to have a certain degree of stability. Here, a new problem has been found that in the case where even a minute amount of copper is mixed in the production stage of an ophthalmic preparation containing delgocitinib, the ophthalmic preparation is colored and the stability thereof is lowered.
An object of the present invention is to provide an aqueous composition with excellent stability even in the case where a minute amount of copper is mixed, containing delgocitinib or a salt thereof as an active ingredient.

### Solution to Problem

As a result of diligent studies to solve the problem, the present inventor has found that by adding edetic acid, creatinine or a salt thereof to an aqueous composition containing delgocitinib and a minute amount of copper, the stability of the aqueous composition is remarkably improved. The present invention is based on the finding and each of the following inventions is provided.

[1] An aqueous composition comprising delgocitinib or a salt thereof (A) and at least one selected from the group consisting of edetic acid, creatinine, and a salt thereof (B).
[2] The aqueous composition according to item [1], wherein the content of the component (A) is 0.003 mass% to 3 mass% based on the total amount of the aqueous composition.
[3] The aqueous composition according to item [1] or [2], wherein the content of the component (B) is 0.0001 mass% to 1 mass% based on the total amount of the aqueous composition.
[4] The aqueous composition according to any one of items [1] to [3], wherein the aqueous composition is for ophthalmology.

### Advantageous Effect of Invention

According to the present invention, an aqueous composition with excellent stability even in the case where a minute amount of copper is mixed, containing delgocitinib or a salt thereof as an active ingredient, can be provided.

### Description of Embodiments

Hereinafter, embodiments of the present invention will be described in detail. The present invention, however, is not limited to the following embodiments. In the present specification, "mass%" is synonymous with "w/v%".
The aqueous composition according to the present embodiment contains delgocitinib or a salt thereof (A) (also

referred to as "component (A)") and at least one selected from the group consisting of edetic acid, creatinine, and a salt thereof (B) (also referred to as "component (B)").

[Component (A)]

Delgocitinib is also referred to as 3-[(3S,4R)-3-methyl-6-(7H-pyrrolo[2,3-d]pyrimidin-4-yl)-1,6-diazaspiro[3,4]octan-1-yl]-3-oxopropanenitrile, which is a known compound represented by the following formula:

[Chemical Formula 1]

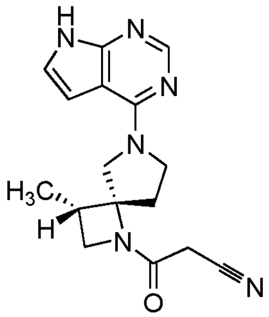

Delgocitinib or a salt thereof may be produced, for example, by the method described in International Publication No. WO 2017/006968 or International Publication No. WO 2018/117151.

The salt of delgocitinib is not particularly limited as long as it is pharmaceutically, pharmacologically (in drug manufacturing) or physiologically acceptable. Specific examples of the salts include salts with an inorganic acid, salts with an organic acid, salts with an inorganic base, salts with an organic base, salts with an acidic amino acid, and salts with a basic amino acid.

Examples of the salts with an inorganic acid include salts with hydrochloric acid, hydrobromic acid, sulfuric acid, nitric acid, or phosphoric acid. Examples of the salts with an organic acid include salts with acetic acid, succinic acid, fumaric acid, maleic acid, tartaric acid, citric acid, lactic acid, stearic acid, benzoic acid, methanesulfonic acid (mesylic acid), ethanesulfonic acid, or p-toluenesulfonic acid. Examples of the salts with an inorganic base include alkali metal salts such as a sodium salt and a potassium salt, alkaline earth metal salts such as a calcium salt and a magnesium salt, and an aluminum salt and an ammonium salt. Examples of the salts with an organic base include salts with diethylamine, diethanolamine, meglumine, or N,N-dibenzylethylenediamine. Examples of the salts with an acidic amino acid include salts with aspartic acid, or glutamic acid. Examples of the salts with a basic amino acid include salts with arginine, lysine, or ornithine.

The aqueous composition according to the present embodiment contains delgocitinib or a salt thereof as an active ingredient, and may be used for the treatment of corneal epithelium disorder caused by an intrinsic disease such as dry eye (xerophthalmia syndrome), Sjogren's syndrome, and Stevens-Johnson syndrome, or corneal epithelium disorder caused by exogenous diseases resulting from post-operation, drug-induction, external injury, or contact lens wearing.

The aqueous composition according to the present embodiment promotes tear secretion due to containing delgocitinib or a salt thereof, and therefore may be used for improving dry eye symptoms. The dry eye may be a dry eye caused by autoimmune diseases such as Sjogren's syndrome, or may be a dry eye caused by a factor other than autoimmune diseases.

The content of the component (A) in the aqueous composition according to the present embodiment is not particularly limited, and is appropriately set according to the type and content of other compounding components, formulation form, and the like. From the viewpoint of exerting the effect of the present invention more remarkably, the content of the component (A) is as follows. For example, based on the total amount of the aqueous composition according to the present embodiment, the total content of the component (A) may be 0.003 mass% to 3 mass%, 0.005 mass% to 1 mass%, 0.01 mass% to 0.5 mass%, 0.015 mass% to 0.4 mass%, or 0.03 mass% to 0.3 mass%.

[Component (B)]

Edetic acid, creatinine, or a salt thereof as component (B) is not particularly limited as long as it is pharmaceutically, pharmacologically (in drug manufacturing) or physiologically acceptable.

Edetic acid is also referred to as ethylenediaminetetraacetic acid (EDTA), which is a known compound represented by $C_{10}H_{16}N_2O_8$.

Examples of the salt of edetic acid include an alkali metal salt such as sodium edetate, disodium edetate, and tetrasodium edetate. As edetic acid or a salt thereof, disodium edetate is preferred. Edetic acid and the salt thereof may be a hydrate or an anhydride. Edetic acid or the salt thereof may be used alone or in combination of two or more.

Creatinine is also referred to as 2-amino-1-methyl-2-imidazolin-4-one or 2-imino-1-methylimidazolidin-4-one, which is a known compound represented by $C_4H_7N_3O$.

Examples of the salt of creatinine include an inorganic acid salt such as creatinine hydrochloride. As creatinine or a salt thereof, creatinine is preferred. Creatinine and a salt thereof may be a hydrate or an anhydride. Creatinine or a salt thereof may be used alone or in combination of two or more.

The content of the component (B) in the aqueous composition according to the present embodiment is not particularly limited, and set appropriately according to the type and content of other compounding components, the formulation form, etc. From the viewpoint of exerting the effect of the present invention more remarkably, the content of the component (B) is as follows. For example, the total content of the component (B) based on the total amount of the aqueous composition according to the present embodiment may be 0.00001 mass% or more, 0.00005 mass% or more, 0.0001 mass% or more, or 0.0005 mass% or more, and may be 0.05 mass% or less, 0.04 mass% or less, 0.03 mass% or less, or 0.02 mass% or less. Alternatively, the content of the component (B) may be 0.000001 mass% to 10 mass%, 0.00001 mass% to 1 mass%, 0.0001 mass% to 0.1 mass%, 0.0005 mass% to 0.05 mass%, 0.0001 mass% to 0.05 mass%, 0.00001 mass% to 0.1 mass%, or 0.00001 mass% to 0.05 mass%.

The content ratio of the component (B) to the component (A) in the aqueous composition according to the present embodiment is not particularly limited, and appropriately set depending on the type of the component (B), the type and content of other compounding components, the formulation form, etc. From the viewpoint of further enhancing the effect of the present invention, the content ratio of the component (B) to the component (A) is as follows. For example, the total content of the component (B) relative to 1 part by mass of the total content of the component (A) in the aqueous composition according to the present embodiment may be 0.0000003 parts by mass to 4000 parts by mass, 0.00001 parts by mass to 200 parts by mass, 0.0002 parts by mass to 10 parts by mass, or 0.001 parts by mass to 4 parts by mass.

[Buffer]

The aqueous composition according to the present embodiment may further contain a buffer. The aqueous composition further containing a buffer allows the effect of the present invention to be more remarkably exhibited. The buffer is not particularly limited as long as it is pharmaceutically, pharmacologically (in drug manufacturing) or physiologically acceptable.

The buffer is not limited, and examples thereof include a boric acid buffer (for example, boric acid and a combination of boric acid and borax), a carbonic acid buffer, an acetic acid buffer, a tris buffer, aspartic acid, and an aspartate. As the buffer, a commercially available one may be used. The buffer may be used alone or in combination of two or more. Boric acid is preferred as the buffer.

The content ratio of the buffer to the component (A) in the aqueous composition according to the present embodiment is not particularly limited, and set appropriately according to the type of the component (B) and buffer, the type and content of other compounding components, the use and formulation form of the aqueous composition, etc. From the viewpoint of further enhancing the effect of the present invention, the content ratio of the buffer relative to the component (A) is as follows. For example, the total content of the buffer relative to 1 part by mass of the total content of the component (A) in the aqueous composition according to the present embodiment may be 0.03 parts by mass to 500 parts by mass, 0.1 parts by mass to 250 parts by mass, or 0.3 parts by mass to 150 parts by mass.

The content ratio of the buffer to the component (B) in the aqueous composition according to the present embodiment is not particularly limited, and set appropriately according to the type of the buffer, the type and content of other compounding components, the use and formulation form of the aqueous composition, etc. From the viewpoint of further enhancing the effect of the present invention, the content ratio of the buffer relative to the component (B) is as follows. For example, the total content of the buffer relative to 1 part by mass of the total content of the component (B) in the aqueous composition according to the present embodiment may be 0.01 parts by mass to 1000000 parts by mass, 0.5 parts by mass to 50000 parts by mass, or 2 parts by mass to 6000 parts by mass.

[Inorganic salt]

The aqueous composition according to the present embodiment may further contain an inorganic salt. The aqueous composition further containing an inorganic salt allows the effect of the present invention to be more remarkably exhibited. The inorganic salt is not particularly limited as long as it is pharmaceutically, pharmacologically (in drug manufacturing) or physiologically acceptable.

Examples of the inorganic salts include chloride salts such as sodium chloride, potassium chloride, calcium chloride, and magnesium chloride. Commercially available inorganic salts may be used. As the inorganic salts, one type

may be used alone, or two or more types may be used in combination. As the inorganic salts, sodium chloride and potassium chloride are preferred.

The content of the inorganic salts in the aqueous composition according to the present embodiment is not particularly limited, and set appropriately according to the type of inorganic salt, the type and content of other compounding components, the use and formulation form of the aqueous composition, etc. From the viewpoint of exerting the effect of the present invention more remarkably, the content of the inorganic salts is as follows. For example, the total content of the inorganic salts based on the total amount of the aqueous composition may be 0.00001 mass% to 3 mass%, 0.0001 mass% to 2 mass%, or 0.001 mass% to 1.5 mass%.

The pH of the aqueous composition according to the present embodiment is 5.0 to 6.5. With a pH of the aqueous composition controlled to the range, the stability of the aqueous composition containing delgocitinib or a salt thereof as an active ingredient is remarkably improved. From the viewpoint of further remarkably improving the stability of the aqueous composition, the pH of the aqueous composition is preferably 5.0 to 6.0. The pH of the aqueous composition may be 4.0 to 6.0, 4.2 to 5.8, 4.3 to 5.7, or 4.5 to 5.5.

The aqueous composition according to the present embodiment may be adjusted to an osmotic pressure ratio within a range acceptable to a living body on an as needed basis. The suitable osmotic pressure ratio may be appropriately set depending on the use, the formulation form, the usage method, etc. of the aqueous composition, and for example, may be set to 0.4 to 5.0, preferably 0.6 to 3.0, more preferably 0.8 to 2.2, and still more preferably 0.8 to 2.0. The osmotic pressure ratio is the ratio of osmotic pressure of a sample to 286 mOsm (osmotic pressure of 0.9 w/v% sodium chloride aqueous solution) based on Japanese Pharmacopoeia 17th edition, and the osmotic pressure is measured with reference to the osmotic pressure measurement method described in Japanese Pharmacopoeia (cryoscopic method). The standard solution for measuring the osmotic pressure ratio (0.9 w/v% sodium chloride aqueous solution) may be prepared by drying sodium chloride (Japanese Pharmacopoeia standard reagent) at 500 to 650°C for 40 to 50 minutes, then cooling it in a desiccator (silica gel), precisely weighing 0.900 g thereof, and dissolving it in purified water to prepare exactly 100 mL, or by using a commercially available standard solution for measuring osmotic pressure ratio (0.9 w/v% sodium chloride aqueous solution).

The viscosity of the aqueous composition according to the present embodiment is not particularly limited as long as it is pharmaceutically, pharmacologically (in drug manufacturing) or physiologically acceptable. As the viscosity of the aqueous composition according to the present embodiment, for example, the viscosity at 20°C measured with a rotational viscometer (RE550 type viscometer, manufactured by Toki Sangyo Co., Ltd., rotor; 1°34'×R24) is preferably 0.5 to 10 mPa·s, more preferably 1 to 5 mPa·s, and still more preferably 1 to 3 mPa·s.

The aqueous composition according to the present embodiment may be prepared, for example, by adding and mixing a desired content of the component (A), the component (B) and, on an as needed basis, other components. Specifically, for example, the preparation is performed by dissolving or suspending the components in purified water and sterilizing the liquid by filtration sterilization or the like.

The aqueous composition according to the present embodiment may be in various dosage forms depending on the purpose, and examples thereof include liquid preparations, gel preparations, and semi-solid preparations (ointments, etc.).

The aqueous composition according to the present embodiment may be used for ophthalmology. Further, the aqueous composition according to the present embodiment may be used, for example, as eye drops (also referred to as an eye wash or an ophthalmic solution; eye drops include an artificial tear solution and eye drops that can be instilled while wearing contact lenses).

In the case where the aqueous composition according to the present embodiment is an eye drop, the dosage and administration thereof is not particularly limited as long as it is effective and has few side effects, and examples thereof include, for adults (15 years old or older) and children 7 years old or older, 1 drop or 1 to 2 drops in an eye at a time, 4 times a day, and 1 drop or 1 to 2 drops in an eye at a time, 5 to 6 times a day.

## Examples

Hereinafter, the present invention will be specifically described based on test examples, though the present invention is not limited thereto. Unless otherwise specified, the unit of each component in the tables is mass%.

[Example 1: Stability evaluation through visual inspection]

Aqueous compositions were prepared according to a conventional method with the compositions shown in Table 1. The pH of each of the aqueous compositions was controlled to 5.5 with hydrochloric acid and sodium hydroxide. Into three containers made of glass, 5 mL each of the aqueous compositions thus prepared were dispensed, and to each of the dispensed aqueous compositions, copper sulfate pentahydrate was added to a concentration of 4 ppm, and the mixture was left to stand at 60°C for 3 weeks.

Each of the aqueous compositions after standing under the conditions was poured into a colorless test tube (inner diameter: 15 mm, Fisherbrand disposable culture tube, borosilicate glass, 16 × 150 mm (Cat. No. 14-961-31)) to make a 30-mm liquid layer in accordance with the property testing of Japanese Pharmacopoeia 17th edition. Then, four trained evaluators evaluated the degree of coloring of each of the aqueous compositions (n = 3) through visual inspection under a white light source (illuminance: 3000 to 5000 lux (measured value: 4680 lux), using Hiroki Lux Meter FT3424 as a light source). In the evaluation, each of the evaluators gave ratings 0 to 3 based on the following criteria, and the average of the ratings of the four persons was calculated. The results are shown in Table 1.

Rating

0: No coloring is observed on a white background (colorless).
1: Slight coloring is observed on a white background (coloring may be identified when compared side by side with purified water).
2: Coloring is observed on a white background (coloring may be identified without comparing side by side with purified water).
3: Coloring is clearly observed even without a white background.

[Table 1]

| | Test Example | Test Example | Test Example | Test Example | Test Example | Test Example | Test Example | Test Example |
|---|---|---|---|---|---|---|---|---|
| | 1 | 2 | 3 | 4 | 5 | 6 | 7 | 8 |
| Delgocitinib | 0.03 | 0.03 | 0.03 | 0.3 | 0.3 | 0.3 | 0.3 | 0.3 |
| Boric acid | 1.2 | 1.2 | 1.2 | 1.2 | 1.2 | 1.2 | 1.2 | 1.2 |
| Sodium chloride | 0.3 | 0.3 | 0.3 | 0.3 | 0.3 | 0.3 | 0.3 | 0.3 |
| Edetic acid | - | 0.01 | - | - | 0.001 | 0.01 | 0.1 | - |
| Phosphoric acid | - | - | 0.01 | - | - | - | - | 0.01 |
| Purified water | Residual amount | Residual amount | Residual amount | Residual amount | Residual amount | Residual amount | Residual amount | Residual amount |
| Average of rating | 1.3 | 0.0 | 1.5 | 3.0 | 0.2 | 0.5 | 1.3 | 3.0 |

[Example 2: Stability evaluation through absorbance measurement]

For preparation, aqueous compositions were poured into a container made of glass (Test Examples 9 to 14) and a container made of polyethylene (Test Examples 15 to 23) according to a conventional method with the compositions shown in Tables 2 and 3. In Tables 2 and 3, "phosphoric acid" is sodium dihydrogen phosphate and "edetic acid" is sodium edetate. The pH of both aqueous compositions were controlled to 5.5 with hydrochloric acid and sodium hydroxide. After adding copper sulfate pentahydrate to a concentration of 4 ppm to each of the prepared aqueous compositions, the mixture was left to stand at 60°C for 10 days.

Each of the aqueous compositions after standing under the conditions was subjected to measurement of absorbance at 420 nm with a microplate reader. Then, the stability improvement rate of delgocitinib was calculated from the measured absorbance according to the following (formula 1), (formula 2) or (formula 3). The results are shown in Tables 2 and 3. The following (formula 1) was used for Test Examples 10 to 14, the following (formula 2) was used for Test Examples 16 to 17, and the following (formula 3) was used for Test Examples 19 to 23, respectively.

(Formula 1): Stability improvement rate (%) = 100 × {(Absorbance of Test Example 9) – (Absorbance of each test example)} / (Absorbance of Test Example 9)

(Formula 2): Stability improvement rate (%) = 100 × {(Absorbance of Test Example 15) – (Absorbance of each test example)} / (Absorbance of Test Example 15)

(Formula 3): Stability improvement rate (%) = 100 × {(Absorbance of Test Example 18) – (Absorbance of each test example)} / (Absorbance of Test Example 18)

[Table 2]

| | Test Example 9 | Test Example 10 | Test Example 11 | Test Example 12 | Test Example 13 | Test Example 14 |
|---|---|---|---|---|---|---|
| Delgocitinib | 0.3 | 0.3 | 0.3 | 0.3 | 0.3 | 0.3 |
| Boric acid | 1.2 | 1.2 | 1.2 | 1.2 | 1.2 | 1.2 |
| Sodium chloride | 0.3 | 0.3 | 0.3 | 0.3 | 0.3 | 0.3 |
| Edetic acid | - | 0.001 | 0.01 | 0.1 | - | - |
| Creatinine | - | - | - | - | 0.01 | - |
| Phosphoric acid | - | - | - | - | - | 0.01 |
| Purified water | Residual amount | Residual amount | Residual amount | Residual amount | Residual amount | Residual amount |
| Stability improvement rate (%) | - | 99.6 | 98.2 | 97.1 | 37.3 | 0.3 |

[Table 3]

| | Test Example 15 | Test Example 16 | Test Example 17 | Test Example 18 | Test Example 19 | Test Example 20 | Test Example 21 | Test Example 22 | Test Example 23 |
|---|---|---|---|---|---|---|---|---|---|
| Delgocitinib | 0.03 | 0.03 | 0.03 | 0.3 | 0.3 | 0.3 | 0.3 | 0.3 | 0.3 |
| Boric acid | 1.2 | 1.2 | 1.2 | 1.2 | 1.2 | 1.2 | 1.2 | 1.2 | 1.2 |
| Sodium chloride | 0.3 | 0.3 | 0.3 | 0.3 | 0.3 | 0.3 | 0.3 | 0.3 | 0.3 |
| Edetic acid | - | 0.01 | - | - | 0.001 | 0.01 | 0.1 | - | - |
| Creatinine | - | - | - | - | - | - | - | 0.01 | - |
| Phosphoric acid | - | - | 0.01 | - | - | - | - | - | 0.01 |
| Purified water | Residual amount | Residual amount | Residual amount | Residual amount | Residual amount | Residual amount | Residual amount | Residual amount | Residual amount |
| Stability improvement rate (%) | - | 97.6 | -5.9 | - | 98.9 | 98.4 | 97.3 | 43.6 | 2.9 |

It was confirmed that addition of a minute amount of copper to the aqueous composition containing delgocitinib caused coloring, which means lowering of the stability. In contrast, it was confirmed that in the aqueous compositions in Test Examples in which edetic acid or creatinine was blended with the aqueous compositions containing delgocitinib, coloring was suppressed even with addition of a minute amount of copper, which means improvement in the stability. On the other hand, it was confirmed that in the aqueous compositions in Test Examples in which phosphoric acid was blended with the aqueous compositions containing delgocitinib, coloring was observed with addition of a minute amount of copper in comparison with the aqueous composition of Test Examples in which edetic acid or creatinine was added, which means that the stability was hardly improved or deteriorated.

## Claims

1. An aqueous ophthalmic composition comprising delgocitinib or a salt thereof (A) and at least one selected from the group consisting of edetic acid, creatinine, and a salt thereof (B).

2. The aqueous ophthalmic composition according to claim 1, wherein a content of the component (A) is 0.003 mass% to 3 mass% based on a total amount of the aqueous ophthalmic composition.

3. The aqueous ophthalmic composition according to claim 1 or 2, wherein a content of the component (B) is 0.0001 mass% to 1 mass% based on the total amount of the aqueous ophthalmic composition.

**INTERNATIONAL SEARCH REPORT**

International application No.
PCT/JP2021/028441

A. CLASSIFICATION OF SUBJECT MATTER
Int. Cl. A61K31/519(2006.01)i, A61K9/08(2006.01)i, A61K47/18(2006.01)i, A61K47/22(2006.01)i, A61P27/02(2006.01)i
FI: A61K31/519, A61K9/08, A61K47/18, A61K47/22, A61P27/02
According to International Patent Classification (IPC) or to both national classification and IPC

B. FIELDS SEARCHED
Minimum documentation searched (classification system followed by classification symbols)
Int. Cl. A61K31/519, A61K9/08, A61K47/18, A61K47/22, A61P27/02

Documentation searched other than minimum documentation to the extent that such documents are included in the fields searched
Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)
CAplus/REGISTRY/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| X | WO 2015/060208 A1 (JAPAN TOBACCO INC.) 30 April 2015 (2015-04-30), claims, paragraphs [0036], [0037], [0042] | 1-3 |
| A | JP 2011-225626 A (ROHTO PHARMACEUTICAL CO., LTD.) 10 November 2011 (2011-11-10), entire text, all drawings | 1-3 |
| A | WO 2007/007832 A1 (SANTEN PHARMACEUTICAL CO., LTD.) 18 January 2007 (2007-01-18), entire text, all drawings | 1-3 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 11.08.2021 | 24.08.2021 |

| Name and mailing address of the ISA/ | Authorized officer |
|---|---|
| Japan Patent Office<br>3-4-3, Kasumigaseki, Chiyoda-ku,<br>Tokyo 100-8915, Japan | <br><br>Telephone No. |

Form PCT/ISA/210 (second sheet) (January 2015)

**INTERNATIONAL SEARCH REPORT**
Information on patent family members

International application No.
PCT/JP2021/028441

| Patent Documents referred to in the Report | Publication Date | Patent Family | Publication Date |
|---|---|---|---|
| WO 2015/060208 A1 | 30.04.2015 | US 2016/0367556 A1 claims, paragraphs [0045], [0046], [0063]<br>EP 3061455 A1<br>CN 105636590 A<br>KR 10-2016-0065979 A | |
| JP 2011-225626 A | 10.11.2011 | JP 2002-302440 A<br>JP 2008-88192 A<br>JP 2013-163700 A<br>JP 2015-13886 A<br>JP 2016-26198 A | |
| WO 2007/007832 A1 | 18.01.2007 | US 2008/0269353 A1 entire text, all drawings<br>EP 1905453 A1<br>KR 10-2008-0039342 A<br>CN 101222939 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017006968 A
- WO 2018117151 A


### Non-patent literature cited in the description


- Japanese Pharmacopoeia